# EUROPÄISCHE PATENTSCHRIFT

(11) **EP 0 570 354 B1**
(45) Veröffentlichungstag und Bekanntmachung des Hinweises auf die Patenterteilung: **02.09.1998**
(21) Anmeldenummer: 93890097.4
(22) Anmeldetag: 12.05.1993
(51) Int. Cl.: C12Q 1/56, G01N 33/86

(54) **Verfahren zur Bestimmung von Fibrinogen**
Method for determination of fibrinogen
Méthode pour la détermination de fibrinogène

(30) Priorität: 15.05.1992 AT 999/92
(43) Veröffentlichungstag der Anmeldung: 18.11.1993
(73) Patentinhaber: IMMUNO Aktiengesellschaft, A-1221 Wien (AT)
(72) Erfinder: Lang, Hartmut, Dr., A-1230 Wien (AT); Moritz, Berta, Dr., A-1030 Wien (AT)
(74) Vertreter: Weinzinger, Arnulf, Dipl.-Ing.

(56) Entgegenhaltungen:
- EP-A- 0 137 269
- CLINICAL CHEMISTRY. Bd. 29, Nr. 4, April 1983, WASHINGTON, DC Seiten 614 - 617 SIEFRING ET AL. 'Development and analytical performance of a functional assay for fibrinogen on the Du Pont aca analyzer'
- DATABASE WPI Week 7417, Derwent Publications Ltd., London, GB; AN 74-31457V & JP-A-48 093 386 (KOWA CO) 3. Dezember 1973
- JOURNAL OF BIOLOGICAL CHEMISTRY Bd. 265, Nr. 18, 25. Juni 1990, BALTIMORE, MD US Seiten 10693 - 10701 DOYLE ET AL. 'Multiple active forms of thrombin'

## Beschreibung

Die Erfindung betrifft ein Verfahren zur Bestimmung von Fibrinogen durch enzymatische Umwandlung mit Thrombin, Prothrombinfragmenten mit einer "thrombin like activity" oder Mischungen daraus und anschließender Detektion des entstandenen Fibrins bzw. der Fibrinogenspaltprodukte. Weiters umfaßt die Erfindung ein Reagens zur Bestimmung des Fibrinogengehaltes einer gegebenenfalls unverdünnten und gegebenenfalls auch heparinhältigen Plasmaprobe.

Der Bestimmung von Fibrinogen in Plasma kommt im Rahmen der Untersuchung von Gerinnungsstörungen große Bedeutung zu. Zur Bestimmung des Fibrinogens sind sowohl immunologische als auch funktionelle Methoden, wie z.B. Gerinnungstests bekannt. Bei den Gerinnungstests wird der Fibrinogengehalt durch Zeitmessung der Gerinnselbildung ermittelt.

Eine übliche Bestimmungsmethode ist von Clauss (Acta Haemat. 17, 237-246, 1957) beschrieben. Oxalatplasma wird in einem Puffer von pH 7,4 verdünnt und eine konzentrierte Thrombinlösung zugesetzt. Im Anschluß daran wird die Zeit bis zum Gerinnungsendpunkt bestimmt. Die Gerinnungszeit der mit Thrombin umgesetzten Plasmaprobe ist aber nur dann zum Fibrinogengehalt proportional, wenn ein genügend großer Überschuß von Thrombin zu Plasma gewählt wird, um plasmatische Störfaktoren auszuschalten. Als Störfaktor gilt Antithrombin III (AT III), welches vor allem gemeinsam mit Heparin die Thrombinwirkung effektiv hemmt. Heparin ist ein gebräuchliches Antikoagulans und wird als Zusatz bei der Blutabnahme oder als Therapeutikum eingesetzt. Ein Heparingehalt in einer Plasmaprobe kann zu verlängerten Gerinnungszeiten und daher zu einem verfälschten Ergebnis führen.

Die Wirkung von Antithrombin III im Plasma wird hier dank der hohen Thrombinkonzentration und dank der Verdünnung des Plasmas, und damit dank der Verringerung der Konzentrationen an AT III bzw. Heparin, stark verringert. Wenn Plasma nicht verdünnt wird, so ist die Antithrombinwirkung um ein Vielfaches stärker und die Gerinnungszeit verlängert.

Eine Plasmaverdünnung und der damit verbundene zusätzliche Verfahrensschritt bedeutet bei Routineuntersuchungen eine erhebliche Belastung. Neben dem höheren Arbeitsaufwand und der zusätzlichen Hantierung mit potentiell infektiösem Plasma ist jede Verdünnung eine prinzipielle Fehlerquelle. Außerdem müssen Verdünnungen oft entsprechend dem Fibrinogengehalt durchgeführt werden, was das Arbeiten mit Analysenautomaten erschwert.

Üblicherweise wird zur Plasmaverdünnung Veronalpuffer verwendet, da dieser Puffer die Bildung von meßbaren Fibringerinnseln erleichtert, was besonders bei Plasmen mit geringem Fibrinogengehalt die Bestimmung verbessert. Veronal ist aber gleichzeitig auch ein Betäubungsmittel mit Suchtgiftcharakter, weshalb der Umgang mit dieser Substanz möglichst vermieden werden sollte.

Weitere Versuche, den Störfaktor Antithrombin III bzw. Heparin auszuschalten, beinhalten die Neutralisation bzw. das Entfernen des Heparins. In der Clinical Chemistry 29, 614-617 (1983) ist ein Verfahren beschrieben, nach dem Heparin in einer Plasmaprobe durch Zusatz von Polybren neutralisiert wird. Plasma wird in einem Puffer von pH 7,4 verdünnt und nach Zugabe von Thrombin in ein Photometer transferiert. Danach wird die Reaktionsgeschwindigkeit nephelometrisch bestimmt.

Durch den Einsatz von Schlangengiftenzymen mit einer thrombinartigen Wirksamkeit (Batroxobin) anstelle von Thrombin kann Fibrinogen in Plasma unabhängig von den genannten Störfaktoren erfaßt werden. Diese Schlangengiftenzyme werden nämlich nicht durch AT III bzw. Heparin inhibiert.

Die Verwendung von Proteinen, welche nicht als natürliche Gerinnungsenzyme gelten, wie z.B. Schlangengiftenzyme, in Verfahren zur Bestimmung von humanen Proteinen ist problembehaftet. Die Reaktionen zwischen einem nicht-Säugetier-Enzym und einem humanen Protein sind oft unspezifisch. Entsprechend dem Prinzip der biochemischen Analytik, nur Gleiches mit Gleichem zu vergleichen und zu untersuchen, wird danach getrachtet, zur Bestimmung von humanen Proteinen ebenfalls nur Enzyme von Säugetieren und v.a. humane Enzyme als Reaktionspartner zu verwenden.

Thrombin kann durch Aktivierung von Prothrombin hergestellt werden. Als Vorstufen entstehen während der Aktivierung Meizothrombin (MT) und Meizothrombin (desF1). Die zweikettigen Thrombinzwischenstufen weisen aber im Gegensatz zu Prothrombin eine Proteaseaktivität auf, die der des Thrombins ähnlich ist ("thrombin like activity"). Sie sind daher in der Lage, Substrate, die von Thrombin umgesetzt werden können, ebenfalls zu spalten. Eine "thrombin like activity" ist also eine Proteaseaktivität, die Thrombinsubstrate zu spalten vermag, deren Reaktionskinetik und Reaktionsparameter, wie pH- oder Ionenstärkeoptima, Inhibitoren, Effektoren, allosterische Wechselwirkungen, usw. aber nicht unbedingt denen von Thrombin gleichen müssen.

Die Aktivität der bovinen Enzyme Meizothrombin, Meizothrombin (desF1) und Thrombin werden im J. of Biol. Chemistry 265, 10693-10701 (1990) bei einem pH von 7,4 verglichen. Meizothrombin zeigt nur 2 % der "fibrinogen clotting activity" von Thrombin. Diese geringe Aktivität kann jedoch auch auf einen 10 bis 15 %igen Gehalt an MT(desF1) in der MT-Zusammensetzung zurückgeführt werden. Für MT(desF1) wurde die Aktivität von 14 % der von Thrombin gefunden.

Der Test zur enzymatischen Umwandlung von Fibrinogen in Fibrin wird üblicherweise bei einem pH-Wert im Bereich von 7,4 bis 8,0 durchgeführt. Dieser pH-Bereich wird bei enzymatischen Reaktionen von Thrombin im allgemeinen verwendet, entspricht dieser doch dem physiologischen pH-Bereich. Der optimale pH-Wert für Thrombinaktivität liegt nahe bei pH 8.

Die Erfindung stellt sich die Aufgabe, ein Verfahren zur Bestimmung von Fibrinogen mittels eines Gerinnungstests zur Verfügung zu stellen, welches die geschilderten Nachteile der bekannten Verfahren vermeidet und insbesondere auch bei unverdünnten Plasmaproben unabhängig vom AT III- bzw. Heparingehalt durchgeführt werden kann.

Diese Aufgabe wird erfindungsgemäß gelöst durch ein Verfahren zur Bestimmung von Fibrinogen weitgehend unabhängig von Thrombininhibitoren durch enzymatische Umwandlung mit Thrombin, Meizothrombin, Meizothrombin (desF1) oder Mischungen daraus und anschließender Detektion des entstandenen Fibrins bzw. der Fibrinogenspaltprodukte, welches dadurch gekennzeichnet ist, daß die enzymatische Umwandlung des Fibrinogens bei einem pH im Bereich von 4 bis 7,3 durchgeführt wird und die Detektion des entstandenen Fibrins durch Bestimmung der Gerinnungszeit bzw. der Fibrinogenspaltprodukte erfolgt. Anschließend wird entstandenes Fibringerinnsel detektiert. Die Detektion kann über die Bestimmung der Gerinnungszeit photometrisch oder turbidimetrisch erfolgen. Die Prothrombinfragmente Meizothrombin, Meizothrombin (desF1) oder Mischungen daraus weisen eine "thrombin like activity" auf. Die Konzentrationen bzw. die Mischungsverhältnisse der eingesetzten Enyzme sind so zu wählen, daß eine lineare Beziehung über einen möglichst weiten Bereich des Fibrinogengehaltes in einer Probe gewährleistet ist.

Auch gentechnisch hergestellte Proteine, welche aufgrund ihrer Struktur eine Aktivität ähnlich der von Thrombin zeigen und im Vergleich zu Thrombin eine geringere Affinität zu Antithrombin III aufweisen, sind für das erfindungsgemäße Verfahren geeignet.

Die Durchführung des Bestimmungsverfahrens im angegebenen pH-Bereich macht dieses von den genannten Störfaktoren AT III und Heparin so weit unabhängig, daß die Fibrinogenbestimmung in einfacher Weise aus einer unverdünnten und gegebenenfalls auch heparinhältigen Plasmaprobe vorgenommen werden kann. Der zusätzliche Verfahrensschritt der Verdünnung von Plasma kann bei dem erfindungsgemäßen Verfahren ausgelassen werden, wodurch das Testergebnis durch Ausschalten einer eventuellen Fehlerquelle verbessert wird.

Die Erfindung beruht auf der Erkenntnis, daß einerseits die Inhibition von Thrombin durch AT III/Heparin bei einem pH von 4 bis 7,3 vernachlässigt werden kann, wobei trotz suboptimaler Bedingungen die Thrombinaktivität zur enzymatischen Umwandlung von Fibrinogen ausreichend ist. Eine etwas verringerte Aktivität von Thrombin hat sogar den Vorteil, daß die Gerinnungszeiten nicht zu kurz und leichter erfaßbar sind.

Bei dem Einsatz von Thrombin, Meizothrombin, Meizothrombin (desF1) oder Mischungen daraus hat sich überraschenderweise herausgestellt, daß die thrombinartige Aktivität in dem genannten pH-Bereich, vorzugsweise bei einem pH von 5 bis 7 oder am meisten bevorzugt bei einem pH von etwa 6, optimal zur Bestimmung von Fibrinogen unabhängig von Thrombininhibitoren ist.

Die enzymatische Umwandlung des Fibrinogens wird vorzugsweise in einem Puffer mit einer Molarität an Puffersalzen im Bereich von 0,02 bis 0,5 M, am meisten bevorzugt mit 0,1 bis 0,25 M, Puffer durchgeführt. Die Ionenstärke kann gegebenenfalls durch Zusatz von Salzen, wie Natriumchlorid, erhöht werden. Es hat sich herausgestellt, daß Thrombin vor allem bei geringen Ionenstärken gegenüber der AT III-Inhibition unempfindlich ist. Für die Durchführung des erfindungsgemäßen Verfahrens ist aber auch ein Milieu mit einer im Vergleich zur Plasmaprobe gleichen oder höheren Pufferkapazität praktikabel, um einen bestimmten pH während des Bestimmungsverfahrens einzuhalten.

Vorteilhaft erfolgt die Fibrinogenbestimmung aus einer unverdünnten und gegebenenfalls auch heparinhältigen Plasmaprobe, in welcher Heparin also in nicht neutralisierter Form vorliegt.

Die Erfindung betrifft auch ein Reagens zur Bestimmung des Fibrinogengehaltes weitgehend unabhängig von Thrombininhibitoren einer gegebenenfalls auch heparinhältigen Plasmaprobe, enthaltend Thrombin, Meizothrombin, Meizothrombin (desF1) oder Mischungen daraus zur enzymatischen Umwandlung von Fibrinogen in einem Puffer mit einem pH im Bereich von 4 bis 7,3 und anschließende Detektion des entstandenen Fibrins durch Bestimmung der Gerinnungszeit bzw. der Fibrinogenspaltprodukte. Das Reagens dient zur einfachen Bestimmung von Fibrinogen, ohne die umständliche Verdünnung von Plasmaproben oder Neutralisierung bzw. Beseitigung von Heparin aus den Plasmaproben in Kauf nehmen zu müssen.

Die Erfindung wird durch die nachfolgenden Beispiele noch näher erläutert.

Bestimmung von Fibrinogen bei pH 6 und pH 8:

Zum Vergleich wurden Meizothrombin und Thrombin bei pH 6 und pH 8 eingesetzt und die Gerinnungszeiten in Abhängigkeit vom Fibrinogengehalt in Plasmaproben bestimmt. Meizothrombin wurde durch Aktivierung von humanem Prothrombin mit immobilisiertem Ecarin (Fa. Pentapharm) nach der Vorschrift von Stocker und Müller (Thromb. Haemostasis 65 (6), Abstract 855 (1991)) hergestellt. Plasma-hältige Proben mit unterschiedlichem Fibrinogengehalt wurden für die Testungen herangezogen.

50 µl Plasmaprobe wurden mit 100 µl Puffer (0,2 M Tris HCl-Puffer, pH 8 oder 0,2 M Phosphat-Puffer, pH 6) und 150 µl Meizothrombin (15 NIH-analog E/ml) oder Thrombin (10 NIH E/ml, Thrombin Reagent, Fa. Immuno) versetzt, und die Gerinnungszeit bei 37°C mit einem Schnitger-Gross-Koagulometer (Fa. Amelung) bestimmt. Das Bestimmungsverfahren wurde sowohl in Anwesenheit als auch in Abwesenheit von Heparin (1 E/ml) durchgeführt.

Aus den Tabellen ist ersichtlich, daß der Verlauf der Bezugskurven für Fibrinogen bei pH 6 unabhängig vom Heparingehalt in der Probe ist. Durch einen Gehalt an Heparin in der Probe werden jedoch die Gerinnungszeiten bei pH 8 verlängert; u.zw. sowohl für Meizothrombin als auch für Thrombin als Gerinnungsenzym.

| Meizothrombin | | | | |
|---|---|---|---|---|
| | pH 8 | | pH 6 | |
| Fibrinogengehalt (mg/ml) | o.Hep. | m.Hep. | o.Hep. | m.Hep. |
| 310 | 8,0 | 10,6 | 30,4 | 30,1 |
| 238 | 11,1 | 15,1 | 50,7 | 49,1 |
| 94 | 26,0 | 35,5 | 166,4 | 157,5 |
| 62,5 | 35,6 | >250 | >250 | >250 |

| Thrombin | | | | |
|---|---|---|---|---|
| | pH 8 | | pH 6 | |
| Fibrinogengehalt (mg/ml) | o.Hep. | m.Hep. | o.Hep. | m.Hep. |
| 373 | 11,6 | >200 | 18,6 | 17,3 |
| 310 | 16,1 | >200 | 24,1 | 24,1 |
| 238 | 25,6 | >200 | 28,4 | 30,1 |
| 100 | 50,0 | >200 | 85,9 | 89,8 |

## Patentansprüche

1. Verfahren zur Bestimmung von Fibrinogen weitgehend unabhängig von Thrombininhibitoren durch enzymatische Umwandlung mit Thrombin, Meizothrombin, Meizothrombin (desF1) oder Mischungen daraus und anschließender Detektion des entstandenen Fibrins bzw. der Fibrinogenspaltprodukte, dadurch gekennzeichnet, daß die enzymatische Umwandlung des Fibrinogens bei einem pH im Bereich von 4 bis 7,3 durchgeführt wird und die Detektion des entstandenen Fibrins durch Bestimmung der Gerinnungszeit bzw. der Fibrinogenspaltprodukte erfolgt.

2. Verfahren nach Anspruch 1, dadurch gekennzeichnet, daß die enzymatische Umwandlung des Fibrinogens in einem Puffer mit einer Molarität an Puffersalzen im Bereich von 0,02 bis 0,5 M, vorzugsweise 0,1 bis 0,25 M, sowie nach Erhöhung der Ionenstärke durch Zusatz von Salzen, wie NaCl, durchgeführt wird.

3. Verfahren nach Anspruch 1 oder 2, dadurch gekennzeichnet, daß die Fibrinogenbestimmung aus einer unverdünnten und gegebenenfalls auch heparinhältigen Plasmaprobe erfolgt.

4. Reagens zur Bestimmung des Fibrinogengehaltes weitgehend unabhängig von Thrombininhibitoren einer gegebenenfalls unverdünnten und gegebenenfalls auch heparinhältigen Plasmaprobe, enthaltend Thrombin, Meizothrombin, Meizothrombin (desF1) oder Mischungen daraus zur enzymatischen Umwandlung von Fibrinogen und anschließenden Detektion des entstandenen Fibrins über die Gerinnungszeit bzw. der Fibrinogenspaltprodukte in einem Puffer mit einem pH im Bereich von 4 bis 7,3.

## Claims

1. A method of assaying fibrinogen largely independently of thrombin inhibitors by enzymatic conversion with thrombin meizothrombin, meizothrombin (desF1) or mixtures thereof, and subsequent detection of the fibrin or fibrin cleavage products formed, characterised in that the enzymatic conversion of the fibrinogen is carried out at a pH ranging from 4 to 7.3, and the detection of the fibrin formed is effected by determining the coagulation time or the fibrinogen cleavage products.

2. A method according to claim 1, characterised in that the enzymatic conversion of the fibrinogen is carried out in a buffer having a molarity of buffer salts ranging from 0.02 to 0.5 M, preferably 0.1 to 0.25 M, as well as upon increasing the ionic strength by the addition of salts, such as Nacl.

3. A method according to claim 1 or 2, characterised in that the fibrinogen determination is effected from an undiluted and, optionally, also heparin-containing plasma sample.

4. A reagent for assaying the fibrinogen content largely independently of thrombin inhibitors in an optionally undiluted and optionally also heparin-containing plasma sample containing thrombin, meizothrombin, meizothrombin (desF1) or mixtures thereof, for the enzymatic conversion of fibrinogen and the subsequent detection of the fibrin formed via the coagulation time, or of the fibrinogen cleavage products, respectively, in a buffer having a pH ranging from 4 to 7.3.

## Revendications

1. Procédé de détermination du fibrinogène largement indépendant des inhibiteurs de la thrombine par transformation enzymatique au moyen de thrombine, de meizothrombine, de meizothrombine (desF1) ou leurs mélanges et de détection subséquente de la fibrine ou des produits de scission du fibrinogène formés, caractérisé en ce que la transformation enzymatique du fibrinogène s'effectue à un pH de l'ordre de 4 à 7,3 et que la détection de la fibrine formée s'effectue par détermination du temps de coagulation ou des produits de scission du fibrinogène.

2. Procédé selon la revendication 1, caractérisé en ce que la transformation enzymatique du fibrinogène s'effectue dans un tampon ayant une molarité en sels tampons de l'ordre de 0,02 à 0,5 M, de préférence de 0,1 à 0,25 M, ainsi qu'après élévation de la force ionique par addition de sels tels que Nacl.

3. Procédé selon la revendication 1 ou 2, caractérisé en ce que la détermination du fibrinogène s'effectue sur un échantillon de plasma non dilué et contenant éventuellement également de l'héparine.

4. Réactif de détermination de la teneur en fibrinogène de façon largement indépendante des inhibiteurs de la thrombine sur un échantillon de plasma éventuellement dilué et contenant également éventuellement de l'héparine, contenant de la thrombine, de la meizothrombine, de la meizothrombine (desF1) ou leurs mélanges en vue de la transformation enzymatique du fibrinogène et de détection subséquente de la fibrine formée par l'intermédiaire du temps de coagulation ou des produits de scission du fibrinogène dans un tampon ayant un pH de l'ordre de 4 à 7,3.
